# EUROPEAN PATENT SPECIFICATION

(11) **EP 3 731 827 B1**
(45) Date of publication and mention of the grant of the patent: **15.04.2026**
(21) Application number: 18918419.5
(22) Date of filing: 26.12.2018
(51) Int. Cl.: A61K 9/20, A61K 9/28, A61K 31/496

(54) **ORAL PHARMACEUTICAL COMPOSITION COMPRISING POSACONAZOLE**
ORALE PHARMAZEUTISCHE ZUSAMMENSETZUNG UMFASSEND POSACONAZOLE
COMPOSITOINS PHARMACEUTIQUES ORALES COMPRENANT DU POSACONAZOLE

(30) Priority: 28.12.2017 TR 201722493
(43) Date of publication of application: 04.11.2020
(73) Proprietor: Sanovel Ilac Sanayi ve Ticaret A.S., Sisli - Istanbul (TR)
(72) Inventor: TÜRKYILMAZ, Ali, 34460 Istanbul (TR); ZENGINER, Sibel, 34460 Istanbul (TR); ULUSOY BOZYEL, Müge, 34460 Istanbul (TR)
(74) Representative: Bakirci, Utkan Bahri
(86) International application number: PCT/TR2018/050906
(87) International publication number: WO 2019/240698

(56) References cited:
- EP-A1- 3 342 399
- WO-A1-2007/143390
- WO-A1-2017/025292
- WO-A1-2019/220282
- CN-A- 108 066 289
- US-A1- 2003 021 846
- US-A1- 2015 231 081
- G. KRISHNA ET AL: "A new solid oral tablet formulation of posaconazole: a randomized clinical trial to investigate rising single- and multiple-dose pharmacokinetics and safety in healthy volunteers", JOURNAL OF ANTIMICROBIAL CHEMOTHERAPY., vol. 67, no. 11, 24 July 2012 (2012-07-24), GB, pages 2725 - 2730, XP055658616, ISSN: 0305-7453, DOI: 10.1093/jac/dks268

## Description

### Field of the invention

The present invention relates to a pharmaceutical composition comprising posaconazole in the form of the free base or in the form of pharmaceutically acceptable salts, crystalline polymorph, solvates, hydrates or esters thereof and a dispersion carrier, wherein the dispersion carrier in the granule is hydroxypropyl methylcellulose acetate succinate (HPMC-AS) and the total amount of HPMC-AS in the granule is between 42.0% and 65.0% by weight of granule.

Furthermore, the composition is obtained using an effective process.

### Background of the Invention

Posaconazole is a derivative of itraconazole and belongs to the second-generation triazole antifungal agents. The IUPAC name of posaconazole is 4-[4-[4-[4-[[(3R,5R)-5-(2,4-difluorophenyl)-5-(1,2,4-triazol-1-ylmethyl)oxolan-3-yl]methoxy]phenyl]piperazin-1-yl]phenyl]-2-[(2S,3S)-2-hydroxypentan-3-yl]-1,2,4-triazol-3-one. Its chemical structure, shown below (Formula I), has a formula of C₃₇H₄₂F₂N₈O₄, with the molecular weight of 700.792 g/mol.

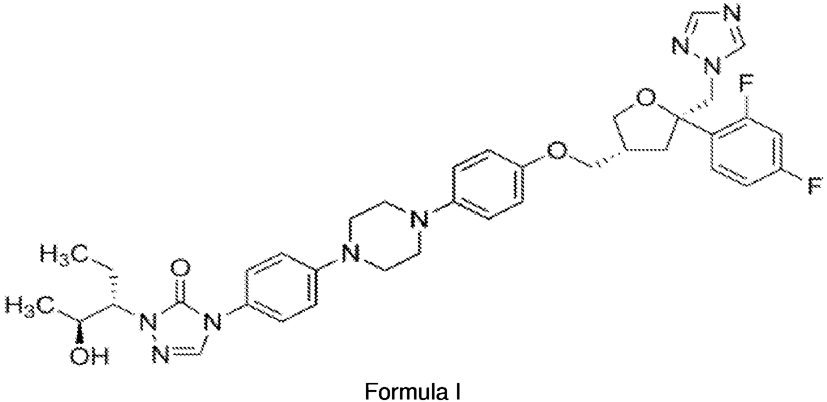

Posaconazole is a triazole antifungal agent indicated for the treatment of invasive fungal infections. US5703079 and US5661151 disclose posaconazole molecule. Three crystalline polymorph forms of posaconazole are form I, II and III. They have been described in patent application numbered WO9918097. Posaconazole is marketed by Merck Sharp & Dohme under the trademark Noxafil^{®}.

In the prior art, there are also several patents which disclose posaconazole in oral pharmaceutical dosage forms. However, many of the formulations in the art rely on complex formulation which can add to the cost of the manufacture of the drug and can be subject to malfunction leading to inappropriate administration of the drug.

There still remains a need in the art to provide an improved oral pharmaceutical composition of posaconazole, having high solubility, dissolution rate, and accordingly a high bioavailability and a long-term stability which is also obtained by using an effective process.

### Detailed Description of the Invention

The main object of the present invention is to provide high solubility, high bioavailability, high physical and chemical stability and a long shelf life by the help of the selection of excipients in a certain ratio using an effective process.

The term "posaconazole" as used herein refers to posaconazole in the form of the free base or in the form of pharmaceutically acceptable salts, crystalline polymorph, solvates, hydrates, esters or mixture thereof.

According to this invention, the pharmaceutical tablet comprises granules and at least one pharmaceutically acceptable excipient, wherein the granules comprise posaconazole and dispersion carrier; wherein the dispersion carrier in the granule is hydroxypropyl methylcellulose acetate succinate (HPMC-AS) and the total amount of HPMC-AS in the granule is between 42.0% and 65.0% by weight of granule.

According to one embodiment of this invention, the total amount of posaconazole in the granule is between 5.0% and 43.0%, between 10.0% and 35.0%, more preferably between 11.0% and 30.0% and between 12.0% and 25.0% by weight of the total composition.

Posaconazole is poorly soluble in water. In the stomach, posaconazole has a solubility of about 0.8 mg/ml. At pH 6.4 or higher, the solubility of posaconazole is less than 1 µg/ml. The solubility of crystalline polymorph forms is higher compared to the solubility of amorphous forms. Thus, it would be desirable to have posaconazole available in crystalline polymorph forms (form I, form II, form III) preferably is in crystalline polymorph form I.

US 2015/231081 A1 discloses delayed release compositions of posaconazole comprising a polymer. US '081 refers to the poor solubility of posaconazole and suggested solution involves posaconazole compositions which are prepared by hot-melt extrusion. The polymer is mentioned to be other than hydroxypropyl methylcellulose derived polymer.

US 2003/021846 A1 discloses an oral dosage form comprising an active ingredient and a polymeric mixture of polyvinyl acetate and polyvinylpyrrolidone. The object of US 2003/021846 A1 is to provide a delayed release formulation. Various groups of active agents are disclosed, the preferred active agents are caffeine, tramadol, diltiazem and propranolol. Krishnal et. al, "A new solid oral tablet formulation of posaconazole: a randomized clinical trial to investigate rising single- and multiple-dose pharmacokinetics and safety in healthy volunteers", J Antimicrob Chemother 2012; 67: 2725-2730, discloses posaconazole tablets which contained 100 mg of posaconazole in a solid dispersion formed by dissolving posaconazole in a pH-sensitive polymer matrix using hot-melt extrusion technology. The drug substance was mixed with hypromellose acetate succinate and ascorbic acid (1:3:0.08, w/w/ w) for the hot-melt extrusion process. Tablets were made from the resulting solid dispersion powder and mixed with small amounts of excipients.

As used herein, 'particle size' means the cumulative volume size distribution as tested by any conventionally accepted method such as the laser diffraction method (i.e. Malvern analysis). The term d (0.1) means, the size at which 10% by volume of the particles are finer and d (0.5) means the size at which 50% by volume of the particles are finer and d (0.9) means the size at which %90 by volume of the particles are finer.

Laser diffraction measures particle size distributions by measuring the angular variation in intensity of light scattered as a laser beam passes through a dispersed particulate sample. Large particles scatter light at small angles relative to the laser beam and small particles scatter light at large angles.The angular scattering intensity data is then analyzed to calculate the size of the particles responsible for creating the scattering. The particle size is reported as a volume equivalent sphere diameter.

The compositions subjected to the invention comprise micron-sized particles of posaconazole in order to overcome the recognized problems in prior art. This invention provides surprisingly better dissolution rate.

According to one embodiment of the present invention, posaconazole has a d (0.1) particle size which is less than 50 µm, less than 40 µm, less than 30 µm, less than 25 µm, less than 20 µm, less than 10 µm.

According to another embodiment of the present invention, posaconazole has a d (0.5) particle size which is less than 60 µm, less than 50 µm, less than 40 µm, less than 30 µm, less than 25 µm, less than 20 µm, less than 10 µm.

According to another embodiment of the present invention, posaconazole has a d (0.9) particle size which is less than 70 µm, less than 60 µm, less than 50 µm, less than 40 µm, less than 30 µm, less than 25 µm, less than 20 µm, less than 10 µm.

According to another embodiment of the present invention, posaconazole has a d (0.1) particle size is less than 50 µm, d (0.5) particle size is less than 60 µm, d (0.9) particle size is less than 70 µm.

At the present invention, the total amount of the granules in the composition is between 40% and 80%, preferably between 55% and 70%, more preferably between 58% and 68% by weight of the total composition.

At the present invention, the composition comprises posaconazole dissolved or molecularly dispersed in a dispersion carrier, which is hydroxypropyl methylcellulose acetate succinate (HPMC-AS).

According to one embodiment of the present invention, the dispersion carrier may have enteric polymer properties. For example; copolymer of ethyl acrylate or methyl methacrylate (Eudragit L100-55), hydroxypropylcellulose-acetate succinate (HPMC-AS) and hydroxypropyl cellulose are enteric polymers.

In this invention, the dispersion carrier is HPMC-AS polymer, in other words, hydroxypropylmethylcellulose-acetate succinate polymer. Furthermore, HPMC-AS polymer may be selected from grade high fine (HF), moderate fine (MF) and low fine (LF). The total amount of HPMC-AS in the granule is between 42.0% and 65.0%, between 43.0% and 55.0% by weight of granule.

According to one embodiment of the present invention, posaconazole is dissolved in HPMC-AS. It has been surprisingly found that a stable and homogenous solution of posaconazole may be prepared with HPMC-AS to enhance the physical and chemical stability of posaconazole in the present composition.

According to one embodiment of the present invention, the ratio of posaconazole to HPMC-AS is in the range of between 4:1 to 1:4 by weight, preferably between 3:1 to 1:3 by weight. This ratio is important in order to provide stability in the present composition.

According to another embodiment of the present invention, the dispersion carrier is copolymer of ethyl acrylate or methyl methacrylate (Eudragit L100 55).

According to one embodiment of the present invention, the granules comprise at least one surfactant.

The present composition may comprise any of a variety of anionic, cationic, nonionic, amphoteric, zwitterionic surfactants.

Suitable surfactants are selected from the group comprising alpha tocopherol, docusate sodium, glyceryl monooleate, glyceryl monostearate, macrogol 15 hydroxystearate, phospholipids, poliyoxylglycerides, polyethylene glycol, triacetin, sodium lauryl sulphate, sorbitan esters, potassium cetylphosphate, ethylene glycol distearate, sodium dodecanoate, dioctyl sodium sulfosuccinate, sodium stearate, benzalkonium chlorides, polysorbates, poloxamers, polyoxyethylene castor oil derivatives, bile salts, lecithin, 12-Hydroxystearic acid-polyethylene glycol copolymer, sodium dodecanesulfonate, sodium oleyl sulfate, and sodium laurate, alkyltrimethylammonium bromides or mixtures thereof.

According to one embodiment of the present invention, the total amount of surfactant in the granules is between 0.1% to 5.0% by weight of granule.

In the present invention, the pharmaceutical composition is suitable for oral administration. Dry oral dosage is used for having effective antifungal activity and bioavailability. Suitable dosage forms are selected from the group comprising tablets, capsules, granules, powders, pellet and unit dose packets. In this invention, the composition is in the form of a tablet. In certain embodiments, oral dosage forms have a drug loading capacity of at least 20 mg per oral dosage form.

According to one embodiment of the present invention, the composition further comprises at least one pharmaceutically acceptable excipient which is selected from the group comprising disintegrants, fillers, lubricants, glidants, coating agents or mixtures thereof.

Suitable disintegrants are selected from the group comprising crospovidone, low-substituted cellulose, carboxymethyl cellulose, sodium carboxymethyl cellulose, crosslinked sodium carboxymethyl cellulose, calcium carboxymethyl cellulose, sodium carboxymethyl starch, hydroxymethyl starch or mixtures thereof.

Suitable fillers are selected from the group comprising microcrystalline cellulose, sucrose, starches derived from wheat, corn rice and potato, gelatin and tragacanth, alginic acid, sodium carboxymethylcellulose sodium alginate, ammonium calcium alginate, polyvinylpyrrolidone, methylcellulose, sodium croscarmellose or mixtures thereof.

Suitable lubricants are selected from the group comprising magnesium stearate, calcium stearate, sodium stearyl fumarate, potassium stearate, stearic acid, high melting point waxes, sodium chloride, sodium benzoate, sodium acetate, sodium oleate, polyethylene glycols or mixtures thereof.

Suitable glidants are selected from the group comprising colloidal silicon dioxide, water soluble excipient, hydrophilic polymers, silicon dioxide or mixtures thereof, preferably glidant is colloidal silicon dioxide.

Suitable coating agents are selected from the group comprising polymethacrylates preferably copolymer of ethyl acrylate or methyl methacrylate (Eudragit L100 55), hydroxypropyl methylcellulose, triethyl citrate, lactose monohydrate, hydroxypropyl cellulose, polyvinyl alcohol (PVA), polyethylene glycol (PEG), talc, polyvinyl alcohol-polyethylene glycol copolymers (Kollicoat^{®} IR), ethylcellulose dispersions (Surelease^{®}), polyvinylprolidone, polyvinylprolidone-vinyl acetate copolymer (PVP-VA), all kinds of Opadry^{®}, pigments, dyes, titanium dioxide, iron oxide, water or mixtures thereof.

The selected coating agents may have enteric coating properties. The enteric coating is achieved with the help of enteric polymers on oral administration that prevents active agent's dissolution or disintegration in the gastric environment.

According to another embodiment of the present invention, the pharmaceutical tablet comprises;
40.0-80.0% by weight of granule comprising posaconazole and dispersion carrier; wherein the dispersion carrier in the granule is hydroxypropyl methylcellulose acetate succinate and the total amount of HPMC-AS in the granule is between 42.0% and 65.0% by weight of granule
15.0-38.0% by weight of microcrystalline cellulose
1.0-10.0% by weight of crospovidone
0.01% - 3.0% by weight of magnesium stearate
1.0% - 15.0% by weight of coating agent

According to another embodiment of the present invention, the granule comprises;
5.0-43.0% by weight of posaconazole
42.0-65.0% by weight of HPMC-AS

Optionally, 0.1-5.0% by weight of surfactant

According to one embodiment of the present invention, the process of preparing granule is hot melt extrusion, direct compression, spray drying, wet granulation, dry granulation or mixtures thereof. Preferably it is spray drying or hot melt extrusion.

According to one embodiment of the present invention, granules is obtained by spray drying which is a method of producing a dry powder from a liquid by rapidly drying with a hot gas. This is the preferred method for drying many pharmaceuticals. The method provides desired stability in the tablet. Solvent is used in the process.

Suitable solvents are selected from the group comprising dichloromethane, 0.1N HCl, methanol, ethanol, isopropyl alcohol, benzyl alcohol, propylene glycol, polyethylene glycol, glycerine, cyclomethicone, glycerine triacetate, diethylene glycol monoethyl ether, glycerol formal, propylene carbonate or mixtures thereof.

The pharmaceutical tablet comprises 40.0-80.0% by weight of granule, 15.0-38.0% by weight of filler, 1.0-10.0% by weight of disintegrant, 0.01% - 3.0% by weight of lubricant, 1.0-15.0% by weight of coating agent. The formulation is produced using spray drying method.

The process for preparing the pharmaceutical tablet processed spray drying comprise the following steps:
a) mixing posaconazole, at least one polymer, optionally surfactant
b) dissolving the mixture in dichloromethane or HCl or ethanol or methanol or mixtures thereof
c) drying the mixture using spray drying method
d) obtaining granules
e) mixing the granules, filler and disintegrant until homogeneous
f) adding lubricant and glidant and mixing
g) then, pressing the mixture into tablets
h) coating the tablets with a coating

According to another embodiment of the present invention, granules are obtained that at the end of the hot melt extrusion, extrudates form is converted to granules.

According to another embodiment of the present invention, a d (0.9) particle size of obtained extrudate using hot melt extrusion is less 250 µm, more preferably it is less 200 µm. The size of the extrudate provides surprisingly better solubility and stability.

Hot-melt extrusion (HME) technology is prominent in the pharmaceutical industry. HME offers the potential of shorter and more efficient times to the final product, through reduction of the processing steps involved. HME is used to disperse active agent in a matrix at the molecular level, thus forming solid solutions. This method is used for poorly soluble active agent and desired dissolution rate and stability are obtained.

Hot-melt extrusion is a technique for manufacturing amorphous solid dispersions in which the active agent is melted or dissolved within a dispersion carrier and mixed to produce and stabilize. Functional excipients, such as surfactants, are often added to further aid in processability or improve the dissolution rate of the formulation.

In addition, the modular concept of the individual screw elements and use of different heating zones along the process barrel allows individual adaptation of the processing section to different product and formulation requirements.

According to one embodiment of the present invention, the below-described heating temperatures are used at zones during the hot melt extrusion. The temperature is chosen according to the properties of the dispersion carriers and posaconazole.

**Table 1: The temperatures of the zones during the hot melt extrusion**

| **Zone 1** | **Zone 2** | **Zone 3** | **Zone 4** | **Zone 5** | **Zone 6** | **Zone 7** | **Zone 8** |
|---|---|---|---|---|---|---|---|
| 20-80 °C | 40-100 °C | 70-140 °C | 80-150 °C | 85-155 °C | 90-160 °C | 95-165 °C | 100-170 °C |

Preferably heating is limited to provide a temperature no greater than the fluxing temperature of the mixture comprising posaconazole, dispersion carriers to insure homogeneity of the composition.

Preferably the heating temperature is chosen between 25°C and 60°C at zone 1, between 50°C and 90°C at zone 2, between 80°C and 130°C at zone 3, between 100°C and 145°C at zone 4, between 100°C and 150°C at zone 5, between 110°C and 150°C at zone 6, between 110°C and 155°C at zone 7, between 120°C and 160°C at zone 8.

The temperature ranges given above aresuitable for this invention to provide high solubility, high physical and chemical stability.

Cooling temperature is provided by using glycol at 23°C.

The pharmaceutical tablet comprises 40.0-80.0% by weight of granules, 15.0-38.0% by weight of microcrystalline cellulose, 1.0-10.0% by weight of crospovidone, 0.01% - 3.0% by weight of magnesium stearate, 1.0-15.0% by weight of coating agent. The tablet form of the composition is produced using hot melt extrusion.

The granules comprise 10.0-43.0% by weight of posaconazole, 52.0-75.0% by weight of HPMC-AS, 0.1-5.0% by weight of surfactant.

The process for preparing pharmaceutical tablet processed hot melt extrusion comprises the following steps;
a) dry mixing HPMC-AS, posaconazole and optionally surfactant until a homogeneous mixture is obtained,
b) heating the mixture prepared at step(a) thereby forming a melt,
c) cooling the melt formed at step(b),
d) converting extrudates form to granules
e) mixing filler and disintegrant in separate tank
f) adding the granules into step (e) mixture and mixing
g) then, pressing the mixture into tablets
h) coating the tablets.

In this invention, between steps (b) and (c) the melt is extruded to provide an extrudate with a desired cross-sectional shape.

It is important the feed rate and screw rate value in order to provide homogeneity in the extrudate, also it provides in the composition.

Also, the value of screw rate thus provides minimizing the risk ofthermal degradation of the posaconazole. The following values give the best stability result for the composition.

The temperature of the hot melt extrusion is set in the range of about 40° C to about 160° C and the rotation speed of the screw is set in the range of about 150 to about 280 rpm.

According to one embodiment of the present invention, screw speed during hot melt extrusion process is between 150rpm and 280 rpm. Also, screw speed during hot melt extrusion process is between 150 rpm and 180 rpm, 180 rpm and 220 rpm, 220 rpm and 250 rpm, 250 rpm and 280 rpm.

According to one embodiment of the present invention, feed rate during hot melt extrusion process is between 15 g/min and 30 g/min. Also, feed rate during hot melt extrusion process is between 15 g/min and 18 g/min, 18 g/min and 20 g/min, 20 g/min and 25 g/min, 25 g/min and 28 g/min, 28 g/min and 30 g/min.

According to one embodiment of the present invention, torque value during hot melt extrusion process is between 25% and 27%.

According to another preferred embodiment of the present invention, said formulation is obtained by means of a hot melt extrusion not involving any liquid solvent during the granulation phase.

The compositions of the invention may be developed into tablet comprising immediate release, extended release, sustained release, controlled release, modified release and delayed release or combination thereof. Such compositions may be prepared using rate controlling polymers.

### Example 1: Tablet composition prepared by hot melt extrusion

| | **amount (w/w)** |
|---|---|
| Granules | 40.0% - 80.0% |
| Fillers | 15.0% - 38.0% |
| Disintegrants | 1.0% - 10.0% |
| Lubricants | 0.01% - 3.0% |
| Glidants | 0.01% - 3.0% |
| Coating agents | 2.0% - 13.0% |
| **Total** | **100** |

### Example 2: Tablet composition prepared by hot melt extrusion

| | **amount (w/w)** |
|---|---|
| Granules | 56.0% - 68.0% |
| Microcrystalline cellulose | 27.0% - 38.0% |
| Disintegrants | 2.0% - 10.0% |
| Lubricants | 0.01% - 3.0% |
| Glidants | 0.01% - 3.0% |
| Coating agents | 2.0% - 13.0% |
| **Total** | **100** |

### Example 3: Tablet composition prepared by hot melt extrusion

| | **amount (w/w)** |
|---|---|
| Granules | 56.0% - 70.0% |
| Microcrystalline cellulose | 15.0% - 38.0% |
| Crospovidone | 1.0% - 20.0% |
| Magnesium stearate | 0.01% - 5.0% |
| Colloidal silicon dioxide | 0.01% - 3.0% |
| Coating agents | 2.0% - 10.0% |
| **Total** | **100** |

### Example 4: Tablet composition prepared by spray drying

| | **amount (w/w)** |
|---|---|
| Granules | 40.0% - 78.0% |
| Fillers | 15.0% - 35.0% |
| Disintegrants | 1.0% - 10.0% |
| Lubricants | 0.1% - 3.0% |
| Glidants | 0.01% - 3.0% |
| Coating agents | 2.0% - 15.0% |
| **Total** | **100** |

### Example 5: Tablet composition prepared by spray drying

| | **amount (w/w)** |
|---|---|
| Granules | 50.0% - 70.0% |
| Microcrystalline cellulose | 15.0% - 30.0% |
| Crospovidone | 1.0% - 35.0% |
| Lubricants | 0.1% - 3.0% |
| Glidants | 0.01% - 3.0% |
| Coating agents | 2.0% - 15.0% |
| **Total** | **100** |

### Example 6: Granule composition of the invention

| | **amount (w/w)** |
|---|---|
| Posaconazole | 5.0% - 43.0% |
| Dispersion carrier | 40.0% - 75.0% |
| **Total** | **100** |

### Example 7: Granule composition of the invention comprises 42.0% - 65.0% HPMC-AC

| | **amount (w/w)** |
|---|---|
| Posaconazole | 5.0% - 43.0% |
| HPMC-AS (grade HF, MF, LF) | 40.0% - 75.0% |
| Surfactants | 0.1% - 5.0% |
| **Total** | **100** |

### Example 8: Granule composition (Reference)

| | **amount (w/w)** |
|---|---|
| Posaconazole | 5.0% - 43.0% |
| Eudragit L100-55 | 40.0% - 75.0% |
| Surfactants | 0.1% - 5.0% |
| **Total** | **100** |

### Example 9: Granule composition of the invention comprises 42.0% - 65.0% HPMC-AC

| | **amount (w/w)** |
|---|---|
| Posaconazole | 10.0% - 35.0% |
| HPMC-AS (grade HF, MF, LF) and hydroxypropyl cellulose | 40.0% - 75.0% |
| Surfactants | 0.1% - 5.0% |
| **Total** | **100** |

### Example 10: Granule composition of the invention comprises 42.0% - 65.0% HPMC-AC

| | **amount (w/w)** |
|---|---|
| Posaconazole | 5.0% - 43.0% |
| HPMC-AS (gradeHF,MF,LF) and hydroxypropyl cellulose | 40.0% - 75.0% |
| **Total** | **100** |

Process for the preparation of the granules according to example 1,2,3 comprises;
a) Dry mixing HPMC-AS, posaconazole and optionally surfactant until a homogeneous mixture is obtained,
b) heating the mixture prepared at step(a) thereby forming a melt,
c) cooling the melt formed at step(b),
d) converting extrudate form to granules

Process for the preparation of the tablet composition by hot melt extrusion according to example 1, 2, 3 comprises;
e) mixing filler and disintegrant in a separate tank
f) adding the granules into step (e) mixture
g) Then, pressing the mixture into tablets
h) coating the tablets.

Process for the preparation of the granules according to example 4 and 5 comprises;
a) mixing posaconazole with at least one polymer and optionally a surfactant
b) dissolving the mixture in dichloromethane or HCl or ethanol or methanol or mixtures thereof
c) drying the mixture using spray drying
d) obtaining granules

Process for the preparation of the tablet composition by spray drying according to example 4 and 5 comprises;
e) mixing the granules, filler and disintegrant until a homogeneous mixture is obtained
f) adding magnesium stearate
g) Then, pressing the mixture into tablets
h) Coating the tablets with coating

## Claims

1. A pharmaceutical tablet comprising granules and at least one pharmaceutically acceptable excipient, wherein the granules comprise posaconazole and dispersion carrier; wherein the dispersion carrier in the granule is hydroxypropyl methylcellulose acetate succinate and the total amount of HPMC-AS in the granule is between 42.0% and 65.0% by weight of granule.

2. The pharmaceutical tablet according to claim 1, wherein the total amount of posaconazole in the granules is 5.0% and 43.0% by weight of the total composition.

3. The pharmaceutical tablet according to claim 1 or 2, wherein the ratio of posaconazole to hydroxypropyl methylcellulose acetate succinate is in the range of between 4:1 to 1:4 by weight, preferably between 3:1 to 1:3 by weight.

4. The pharmaceutical tablet according to claim 1, wherein the dispersion carrier in the granule is copolymer of ethyl acrylate or methyl methacrylate or mixtures thereof.

5. The pharmaceutical tablet according to any preceding claims, wherein the granules comprising at least one surfactant.

6. The pharmaceutical tablet according to any preceding claims, further comprising at least one pharmaceutically acceptable excipient which is selected from the group comprising disintegrants, fillers, lubricants, glidants, coating agents or mixtures thereof.

7. The pharmaceutical tablet according to any preceding claims, the tablet comprising;
40.0-80.0% by weight of granule
15.0-38.0% by weight of microcrystalline cellulose
1.0-10.0% by weight of crospovidone
0.01 % - 3.0% by weight of magnesium stearate
1.0% - 15.0% by weight of coating agent .

8. The pharmaceutical tablet according to any of the preceding claims, wherein the granules are obtained at the end of the hot melt extrusion as an extrudate and said extrudate has d (0.9) particle size less than 200 µm.

9. The pharmaceutical tablet according to any of claims 1-7 wherein granule is obtained by spray drying.

10. A process tor preparing the pharmaceutical tablet according claim 9, comprising the following steps;
a) mixing posaconazole with at least one polymer, optionally a surfactant
b) dissolving the mixture in dichloromethane or HCl or ethanol or methanol or mixtures thereof
c) drying the mixture using spray drying
d) obtaining granule
e) mixing the granule, filler and disintegrant until homogeneous
f) adding magnesium stearate
g) then, pressing to form tablet
h) coating the tablet with coating
wherein the granules are obtained by spray drying.

11. A Process tor preparing the pharmaceutical tablet according to any of claims 1-8, comprising the following steps;
a) dry mixing hydroxypropyl methylcellulose acetate succinate, posaconazole, optionally surfactant until homogeneous,
b) heating the admixture prepared at step(a) thereby forming a melt,
c) cooling the melt formed at step(b),
d) converting extrudate forms to granules
e) mixing microcrystalline cellulose and crospovidone in separate tank
f) adding the granules into step (e) mixture
g) then, pressing to form tablet
h) coating the tablets with coating agents
wherein the granules are obtained by hat melt extrusion.

## Patentansprüche

1. Pharmazeutische Tablette, umfassend Granulate und mindestens einen pharmazeutisch akzeptablen Hilfsstoff, wobei die Granulate Posaconazol und einen Dispersionsträger umfassen; wobei der Dispersionsträger im Granulat Hydroxypropylmethylcelluloseacetatsuccinat ist und die Gesamtmenge an HPMC-AS im Granulat zwischen 42,0 Gew.-% und 65,0 Gew.-%, bezogen auf das Gewicht des Granulats, beträgt.

2. Pharmazeutische Tablette nach Anspruch 1, wobei die Gesamtmenge an Posaconazol in den Granulaten 5,0 Gew.-% und 43,0 Gew.-% der Gesamtzusammensetzung beträgt.

3. Pharmazeutische Tablette nach Anspruch 1 oder 2, wobei das Gewichtsverhältnis von Posaconazol zu Hydroxypropylmethylcelluloseacetatsuccinat im Bereich von 4:1 bis 1:4 liegt, vorzugsweise von 3:1 bis 1:3.

4. Pharmazeutische Tablette nach Anspruch 1, wobei der Dispersionsträger im Granulat ein Copolymer aus Ethylacrylat oder Methylmethacrylat oder Mischungen davon ist.

5. Pharmazeutische Tablette nach einem der vorhergehenden Ansprüche, wobei die Granulate mindestens ein Tensid umfassen.

6. Pharmazeutische Tablette nach einem der vorhergehenden Ansprüche, ferner umfassend mindestens einen pharmazeutisch akzeptablen Hilfsstoff, ausgewählt aus der Gruppe umfassend, Zerfallsmittel, Füllstoffe, Gleitmitteln, Fließregulierungsmittel, Überzugsmitteln oder Mischungen davon.

7. Pharmazeutische Tablette nach einem der vorhergehenden Ansprüche, wobei die Tablette umfasst:
40,0 bis 80,0 Gew.-% Granulat
15,0 bis 38,0 Gew.-% mikrokristalline Cellulose 1,0 bis 10,0 Gew.-% Crospovidon
0,01 bis 3,0 Gew.-% Magnesiumstearat
1,0 bis 15,0 Gew.-% Überzugsmitteln.

8. Pharmazeutische Tablette nach einem der vorstehenden Ansprüche, wobei die Granulate am Ende der Heißschmelzextrusion als Extrudat erhalten werden und das Extrudat eine Partikelgröße d (0,9) von weniger als 200 µm aufweist.

9. Pharmazeutische Tablette nach einem der Ansprüche 1 bis 7, wobei das Granulat durch Sprühtrocknung erhalten wird.

10. Verfahren zur Herstellung der pharmazeutischen Tablette gemäß Anspruch 9, umfassend die folgenden Schritte:
a) Mischen von Posaconazol mit mindestens einem Polymer, gegebenenfalls einem Tensid
b) Auflösen der Mischung in Dichlormethan oder HCl oder Ethanol oder Methanol oder Mischungen davon
c) Trocknen der Mischung durch Sprühtrocknung
d) Gewinnen des Granulats
e) Mischen des Granulats, des Füllstoffs und des Zerfallsmittel bis zur Homogenität
f) Zugabe von Magnesiumstearat
g) Anschließend Pressen zur Tablettenformung
h) Beschichten der Tablette mit einer Überzugsmitteln
wobei das Granulat durch Sprühtrocknung gewonnen wird.

11. Verfahren zur Herstellung der pharmazeutischen Tablette nach einem der Ansprüche 1 bis 8, umfassend die folgenden Schritte:
a) Trockenmischen von Hydroxypropylmethylcelluloseacetatsuccinat, Posaconazol und gegebenenfalls Tensid bis zur Homogenität,
b) Erhitzen der in Schritt (a) hergestellten Mischung, wodurch eine Schmelze gebildet wird,
c) Abkühlen der in Schritt (b) gebildeten Schmelze,
d) Umwandeln der Extrudatformen in Granulate
e) Mischen von mikrokristalliner Cellulose und Crospovidon in einem separaten Behälter
f) Hinzufügen des Granulats zur Mischung aus Schritt (e)
g) Anschließend Pressen zur Tablettenformung
h) Beschichten der Tabletten mit Überzugsmitteln
wobei das Granulat durch Heißschmelzextrusion gewonnen wird.

## Revendications

1. Comprimé pharmaceutique comprenant des granules et au moins un excipient pharmaceutiquement acceptable, dans lequel les granules comprennent du posaconazole et un support de dispersion; dans lequel le support de dispersion dans le granule est l'acétate succinate d'hydroxypropylméthylcellulose et la quantité totale de HPMC-AS dans le granule est comprise entre 42,0 % et 65,0 % en poids du granule.

2. Comprimé pharmaceutique selon la revendication 1, dans lequel la quantité totale de posaconazole dans les granulés est comprise entre 5,0 % et 43,0 % en poids de la composition totale.

3. Comprimé pharmaceutique selon la revendication 1 ou 2, dans lequel le rapport entre le posaconazole et l'acétate succinate d'hydroxypropylméthylcellulose est compris entre 4:1 et 1:4 en poids, de préférence entre 3:1 et 1:3 en poids.

4. Comprimé pharmaceutique selon la revendication 1, dans lequel le support de dispersion dans le granule est un copolymère d'acrylate d'éthyle ou de méthacrylate de méthyle ou de mélanges de ceux-ci.

5. Comprimé pharmaceutique selon l'une quelconque des revendications précédentes, dans lequel les granulés comprennent au moins un tensioactif.

6. Comprimé pharmaceutique selon l'une quelconque des revendications précédentes, comprenant en outre au moins un excipient pharmaceutiquement acceptable choisi dans le groupe comprenant des désintégrants, des charges, des lubrifiants, des agents glissants, des agents d'enrobage ou leurs mélanges.

7. Comprimé pharmaceutique selon l'une quelconque des revendications précédentes, le comprimé comprenant:
40,0 à 80,0 % en poids de granulés
15,0 à 38,0 % en poids de cellulose microcrystalline
1,0 à 10,0 % en poids de crospovidone
0,01 % à 3,0 % en poids de stéarate de magnésium
1,0 % à 15,0 % en poids d'un agent d'enrobage.

8. Comprimé pharmaceutique selon l'une quelconque des revendications précédentes, dans lequel les granulés sont obtenus à la fin de l'extrusion à chaud sous forme d'un extrudat d' e et ledit extrudat a une taille de particules d (0,9) inférieure à 200 µm.

9. Comprimé pharmaceutique selon l'une quelconque des revendications 1 à 7, dans lequel le granulé est obtenu par séchage par atomisation.

10. Procédé de préparation du comprimé pharmaceutique selon la revendication 9, comprenant les étapes suivantes :
a) mélanger le posaconazole avec au moins un polymère, éventuellement un tensioactif
b) dissoudre le mélange dans du dichlorométhane ou du HCl ou de l'éthanol ou du méthanol ou des mélanges de ceux-ci
c) sécher le mélange par séchage par atomisation
d) obtention du granulé
e) mélanger les granulés, la charge et l'agent désintégrant jusqu'à obtention d'un mélange homogène
f) ajouter le stéarate de magnésium
g) puis presser pour former le comprimé
h) enrobant le comprimé d'un enrobage
les granulés étant obtenus par séchage par atomisation.

11. Procédé de préparation du comprimé pharmaceutique selon l'une quelconque des revendications 1 à 8, comprenant les étapes suivantes :
a) mélanger à sec l'acétate succinate d'hydroxypropylméthylcellulose, le posaconazole, éventuellement un tensioactif, jusqu'à obtention d'un mélange homogène,
b) chauffer le mélange préparé à l'étape (a) pour former une masse fondue,
c) refroidir la masse fondue formée à l'étape (b),
d) conversion des formes extrudées en granulés.
e) mélanger la cellulose microcristalline et la crospovidone dans un réservoir séparé
f) ajouter les granulés au mélange de l'étape (e)
g) puis presser pour former le comprimé
h) enrobage des comprimés avec des agents d'enrobage, les granulés étant obtenus par extrusion à chaud.
